# EUROPEAN PATENT APPLICATION

(11) **EP 3 696 257 A1**
(43) Date of publication of application: **19.08.2020**
(21) Application number: 18867207.5
(22) Date of filing: 09.10.2018
(51) Int. Cl.: C12M 1/34, C12M 1/00

(54) **ROE PROCESSING APPARATUS**

(30) Priority: 10.10.2017 JP 2017197211
(71) Applicant: Sinfonia Technology Co., Ltd., Tokyo 105-8564 (JP)
(72) Inventor: MIYASHITA, Yuji, Tokyo 105-8564 (JP); NAKANISHI, Akira, Tokyo 105-8564 (JP); KOTO, Saori, Tokyo 105-8564 (JP)
(74) Representative: TBK
(86) International application number: PCT/JP2018/037514
(87) International publication number: WO 2019/073943

(57) **Abstract**

[Problem]

To provide a fish egg processing apparatus capable of performing predetermined processing on fertilized eggs with higher efficiency, and collecting proper fish eggs.

[Solution]

When a housing recess that houses fish eggs one by one is conveyed to a predetermined processing position, and a capillary is raised and lowered to inject a gene solution, the housing recess is imaged from a direction that intersects a depth direction and a conveying-in direction, and an injection operation by the capillary is controlled by determining, along a possibility determination flow, whether to inject a predetermined substance into each of the fish eggs before processing, based on a previously determined condition.

## Description

### TECHNICAL FIELD

The present invention relates to a fish egg processing apparatus for performing predetermined processing mainly on fish eggs.

### BACKGROUND ART

It is known that the use of small-sized fish eggs such as zebrafish is useful in the technical field in which target substances such as recombinant proteins are produced using genetic engineering techniques by injecting genes into fertilized eggs. For example, if zebrafish are used to obtain a target substance, a gene solution (vector) need to be precisely injected into a spherical fertilized egg having a diameter of 0.9 mm to 1.3 mm. There is a known microinjection technique in which, to prevent damages to fertilized eggs, a gene solution is injected by penetrating the egg membrane of each of the fertilized eggs with an extremely thin needle having a tip with a diameter of several to several dozens of micrometers, and inserting the needle tip into the embryo. In this field, a microinjection work using a manipulator, or the like, to prevent a hand shake due to a manual work or a manual operation is generally employed; however, in the microinjection work, it is difficult to process fertilized eggs in an amount required to acquire the practical quantity of target substances, and the accuracy and the stability of an injection process are also limited. Therefore, various attempts have been made to achieve automation as described in the following prior art.

### PRIOR ART DOCUMENT

### PATENT DOCUMENT

Patent Document 1: Japanese Patent No. 5647005
Patent Document 2: Japanese Patent No. 5823112
Patent Document 3: Japanese Patent No. 5787432

### SUMMARY OF THE INVENTION

### [Technical Problem]

However, in a fish egg, the egg membrane and the embryo are different in specific gravity, and thus, the position of the embryo relative to the egg membrane is eccentric in a vertically lower direction due to gravity. In conventional methods in which a gene is injected into fish eggs arranged on a plate or the like, if an operator visually recognizes the fish eggs from above and below, it is difficult to appropriately observe the insertion depth and the state of the insertion of the gene injection needle, and the state of the gene injection to determine the insertion depth of the gene injection needle, and then to inject the gene. It is also difficult to image and sense the insertion depth of the gene injection needle from the front and back and the side in a conveyance direction of the fish eggs with avoiding the influence of a component serving as an aligning and conveying unit for the fish eggs. The same applies to grasping of the needle insertion state and the insertion state of the gene solution.

Further, extremely thin gene injection needles enabling microinjection are of a disposable type to prevent contamination, but if each of the needles is simply attached to an attachment, the length of the needle and the structure of the attachment are often not manufactured at an accuracy level not affecting the accuracy of the needle insertion depth to the fish eggs, and thus, there is variation in length among the needles. Thus, it is necessary to precisely position a plurality of needle tip positions in an area of about several tens of µm, and then prepare a design and a setting procedure in consideration of the mounting alignment, accumulated tolerances of various components, and the like on the apparatus, which requires a complicated prior setup adjustment of the procedure. This is difficult even for a specific skilled operator, and thus, requires time.

In addition, cells in the above-described fertilized eggs frequently divide, for example, at intervals of about 30 minutes, and thus, it is needed that the fertilized eggs are processed with higher efficiency to rapidly introduce a gene to obtain desired fertilized eggs. That is, the related arts disclosed in the above-described patent documents are based on the assumption of what is called batch processing in which the processing speed is restricted due to the use of a predetermined container, or the like, and thus, fertilized eggs are not always processed in a state suitable for processing. Of course, it is also an important factor to efficiently collect the processed fertilized eggs, and at present, there is also a need for a technique for collecting the fertilized eggs more efficiently than the above-described patent documents.

Specifically, it is essential to convey fertilized eggs with higher efficiency to process the fertilized eggs with high efficiency and perform predetermined processing on the fertilized eggs, and it is also essential to collect the processed fertilized eggs with high efficiency.

Therefore, it is conceivable as one effective way to process and collect fish eggs while the fish eggs are sequentially conveyed by an in-line system.

However, with a careless automation, a gene solution may be injected also to empty eggs without embryos or dead eggs. This takes time due to useless injection, and increases waste of the gene solution. Further, as a result of the dead eggs being mixed with eggs provided to an aging step in the subsequent step, problems such as propagation of microorganisms due to contamination are likely to occur.

In addition, if the injection fails in the gene injection step, many substances other than the target substances are provided to the target substance purification step in the subsequent step, and thus, the target substances easily deteriorate.

The present invention has been made in view of such a point, and a main object of the present invention is to provide a fish egg processing apparatus capable of performing predetermined processing on the above-described fertilized eggs with higher efficiency, and collecting proper eggs.

### SOLUTION TO PROBLEM

The present invention has been made in view of the above-described problems and has employed the following means.

That is, a fish egg processing apparatus according to the present invention includes a processing water tank to which fish eggs are guided, an aligning and conveying unit that conveys, to a predetermined processing position, a housing recess that houses the fish eggs one by one in the processing water tank, a processing unit that raises and lowers an introduction tube to inject a predetermined substance into each of the fish eggs in the housing recess conveyed to the processing position, an image processing unit that images the housing recess from a direction that intersects a depth direction and a conveying-in direction, in association with the processing water tank, and a possibility determination unit that determines whether to inject the predetermined substance into each of the fish eggs before processing, based on an image obtained through the image processing unit and a previously determined condition. An injection operation of the processing unit is controlled based on a determination result of the possibility determination unit.

Thus, even if the in-line system is employed, it is possible to prevent the processing from being performed in an improper state not satisfying the conditions or being performed on an improper fish egg.

In this case, it is effective that the predetermined condition includes at least one or both of conditions that: the housing recess is within a predetermined range with respect to the processing unit; and one egg membrane and one embryonic membrane are detected within a predetermined range in the housing recess.

Alternatively, a fish egg processing apparatus according to the present invention includes a processing water tank to which fish eggs are guided, an aligning and conveying unit that conveys, from a predetermined processing position, a housing recess that houses the fish eggs one by one in the processing water tank, a processing unit that raises and lowers an introduction tube to inject a predetermined substance into each of the fish eggs in the housing recess at the processing position, a sorting unit that determines a conveyance destination according to a quality of each of the fish eggs, an image processing unit that images the housing recess from a direction that intersects a depth direction and a conveying-in direction , in association with the processing water tank, and a pass/fail determination unit that determines whether each of the processed fish eggs is suitable for collection, based on an image obtained through the image processing unit and a previously determined condition. A sorting operation of the sorting unit is controlled based on a determination result of the pass/fail determination unit.

Thus, even if the in-line system is employed, it is possible to remove fish eggs to which the predetermined substance is injected improperly without satisfying the conditions.

In this case, it is effective that the predetermined substance contains pigment when the predetermined substance is injected in the processing unit, and the predetermined conditions includes at least one or two, or all of conditions that: one egg membrane and one embryonic membrane are detected within a predetermined range in the image; the number of pixels of pigment detected in the egg membrane is within a predetermined range; and a ratio of pigment detected in the embryo among the pigment detected in the egg membrane is equal to or higher than a predetermined value.

In the above, it is desirable that the fish egg processing apparatus includes a storage unit that stores the determination result of the determination unit at the processing position for each pitch while the housing recess is pitch-fed, and when the sorting unit located a predetermined number of pitches away from the processing position is controlled, a determination result obtained a predetermined number of pitches before is taken out from the storage unit to control the sorting operation of the sorting unit based on the determination result.

Thus, even if the processing position is separated from the sorting position, the determination process and the sorting process can be performed in parallel.

Further, it is desirably configured such that the aligning and conveying unit includes an alignment board in a gear shape provided with the housing recess in the whole circumference, and the image processing unit sets, as a reference point, a relative position at which a front end of the introduction tube is positioned with respect to the housing recess, and automatically acquires a coordinate of a standardized reference point, based on reference points in a plurality of housing recesses imaged while the alignment board is rotated.

Thus, the influence due to assembling errors of the alignment board is minimized, and even if the predetermined substance is injected with the introduction tube being raised and lowered while the housing recess is pitch-fed in an initially positioned state, it is possible to effectively avoid a situation where poor injection occurs or a needle breaks as a result of contact with the alignment board.

### ADVANTAGEOUS EFFECT OF THE INVENTION

According to the present invention described above, it is possible to provide a fish egg processing apparatus capable of performing predetermined processing on fertilized eggs with higher efficiency, and collecting proper fish eggs.

### BRIEF DESCRIPTION OF THE DRAWINGS

FIG. 1 is a functional block diagram according to an embodiment of the present invention;
FIG. 2 is an explanatory diagram illustrating an entire configuration according to the embodiment;
FIG. 3 is a schematic diagram illustrating a collected-egg conveying unit and describing a configuration thereof according to the embodiment;
FIG. 4 is a schematic diagram illustrating an unnecessary-material separating unit and describing a configuration thereof according to the embodiment;
FIG. 5 is a schematic diagram illustrating a gene injection unit and a processing water tank and describing configurations thereof according to the embodiment;
FIG. 6 is a schematic plan view according to FIG. 5;
FIG. 7 is a diagram for describing an effect according the embodiment;

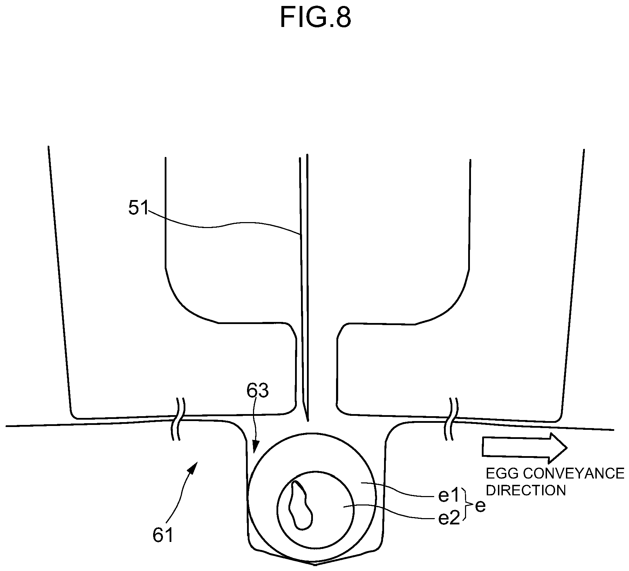

FIG. 8 is an enlarged view of a vicinity of a housing recess according to the embodiment;
FIG. 9 is a block diagram illustrating functions of a control device according to the embodiment;
FIG. 10 is a flowchart illustrating a determination procedure executed by the control device;
FIG. 11 is a flowchart illustrating a control procedure of a sorting unit executed by the control device;
FIGS. 12 are diagrams for describing conditions of possibility determination or pass/fail determination according to the embodiment; and

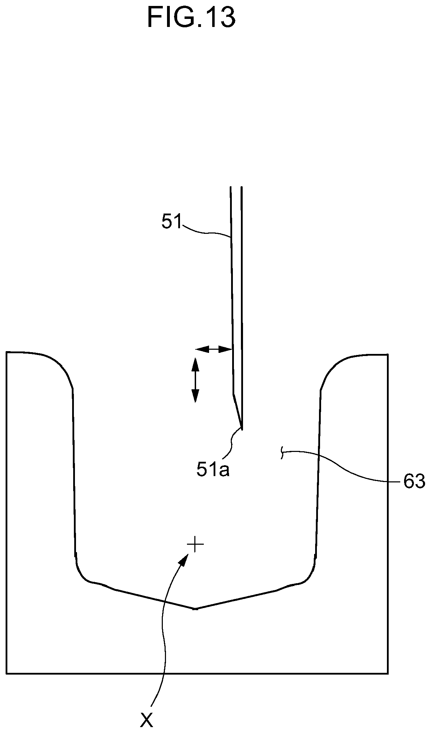

FIG. 13 is a diagram for describing an adjustment step of an introduction tube according to the embodiment.

### DESCRIPTION OF THE EMBODIMENT

An embodiment of the present invention will be described below with reference to the drawings.

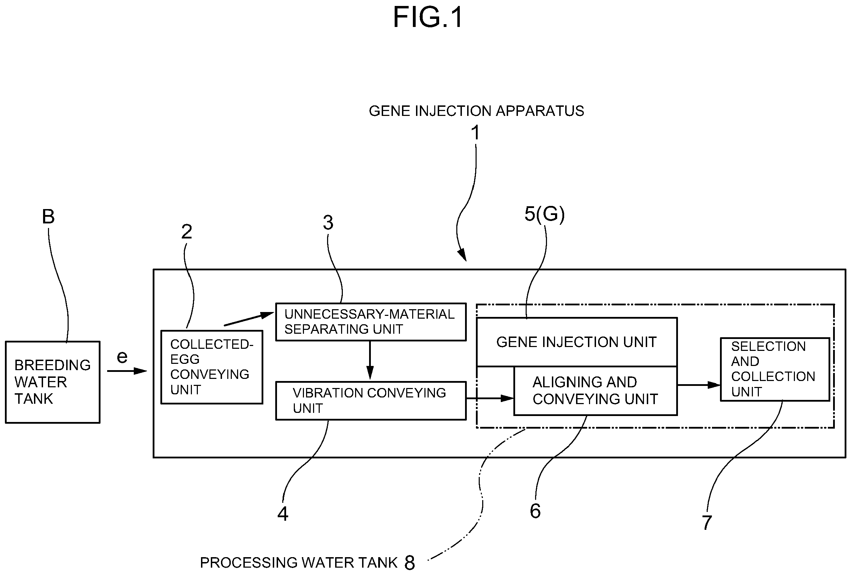

As illustrated in FIG. 1, a gene injection apparatus 1 being an example of a fish egg processing apparatus according to the present embodiment is used to promptly introduce a gene solution G containing a gene, which is a predetermined substance, into each of fertilized eggs, which are fish eggs e collected from a breeding water tank B. In the fish eggs e into which the gene solution G has been injected, cell division is repeated and a protein derived from the base sequence of the introduced gene is synthesized. The protein is collected, extracted, and purified in appropriate timing and is used for, for example, drug development study and mass production.

As illustrated in FIGS. 1 to 4, the gene injection apparatus 1 includes a collected-egg conveying unit 2 that is continuous to the breeding water tank B, an unnecessary-material separating unit 3 that separates conveyed fish eggs e from unnecessary materials bg and sg such as breeding water w, excrement, or remaining food, a vibration conveying unit 4 that conveys, mainly by vibration, the fish eggs e separated from the unnecessary materials bg and sg, an aligning and conveying unit 6 that conveys the fish eggs e conveyed by the vibration conveying unit 4 while arranging the fish eggs e in a row in a predetermined state, a gene injection unit 5 that injects the gene solution G into each of the fish eggs e conveyed by the aligning and conveying unit 6, and a selection and collection unit 7 that efficiently collects the fish eggs e into which the gene solution G has been injected. Further, in the present embodiment, the gene injection unit 5 and the aligning and conveying unit 6, which are processing units included in a processing device for performing predetermined processing on the fish eggs e, and the selection and collection unit 7 serving as a collecting device are placed in a processing water tank 8 having a substantially T-shape in a plan view.

According to the present embodiment, a zebrafish egg having substantially a spherical shape with a diameter of approximately 1 mm is used as an example of the fish egg e. Since fish is a vertebrate, and a protein in the form that can be used for drug development is easily obtained by gene introduction, and in particular, zebrafish is known as the type with which the fish eggs e, which are fertilized eggs, are efficiently obtained from the breeding water tank B.

The configuration of each of components of the gene injection apparatus 1 will be described, below.

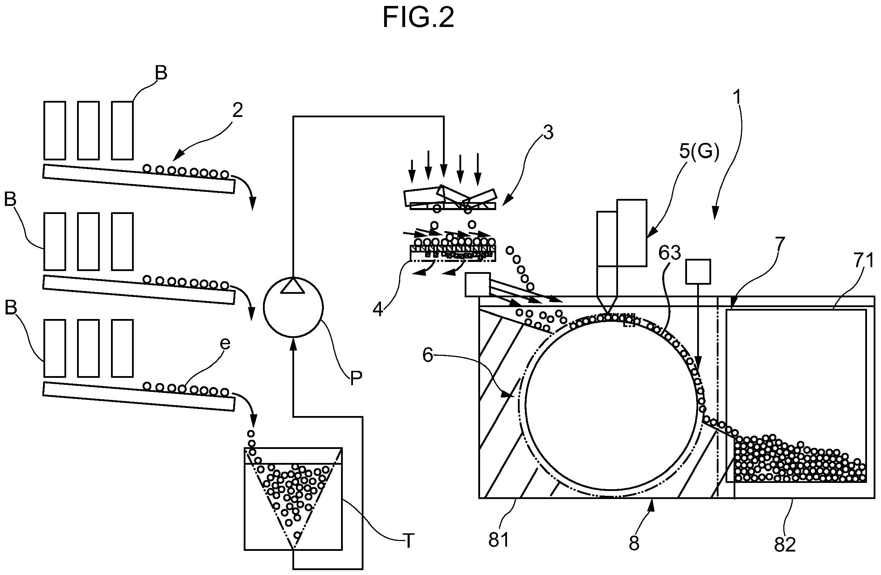

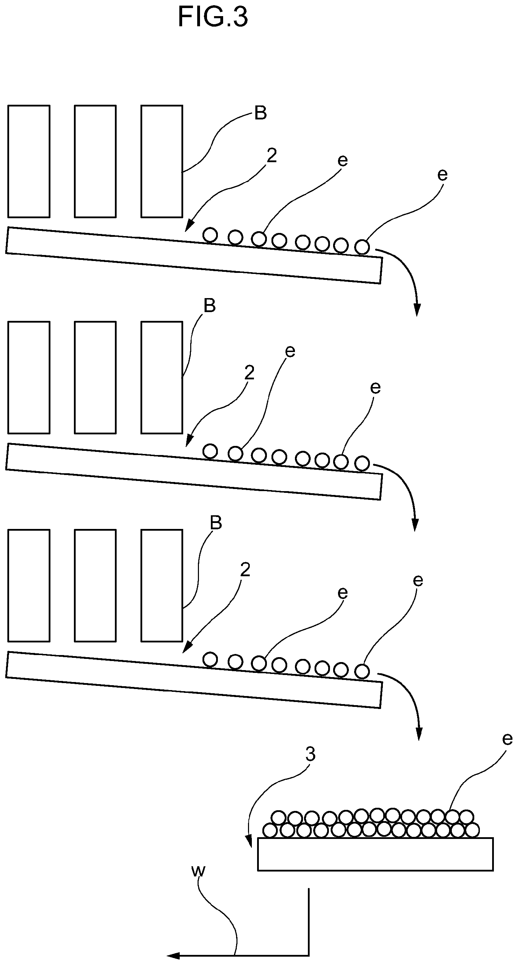

As illustrated in FIGS. 2 and 3, the collected-egg conveying unit 2 is, for example, a water-tank shaped passage configured to have a sloped shape to efficiently collect the fish eggs e from the breeding water tanks B arranged in plurality in parallel and at multiple stages in a vertical direction. The collected-egg conveying unit 2 simultaneously conveys, from the breeding water tanks B, as well as the fish eggs e, the breeding water w in which the fish is bred, and the fish eggs e and the breeding water w are once stored in a tank T and then lifted by a pump P and guided to the unnecessary-material separating unit 3. Specifically, the fish eggs e and the breeding water w fall from above and are guided to the unnecessary-material separating unit 3 in a state where the falling energy is applied. The tank T serves as a temporary cushion tank for the collected and delivered fish eggs e and breeding water w, and in the example of the drawing, can supply water to the pump P without interruption due to the occurrence of, for example, a spiral water flow generated in the tank T. As a specific mode, in the tank T, the lower limit water level is detected to prevent breakage and depletion of the pump P due to idling, and the upper limit water level is detected to detect overflow. If the overflow actually occurs, the breeding water w is returned to the breeding water tank B via a pipe (not illustrated) to the breeding water tank B. If the breeding water tank B can be laid out at a sufficiently high position and the fish eggs e can be freely dropped from the breeding water tank B to the unnecessary-material separating unit 3, the tank T and the pump P may be omitted.

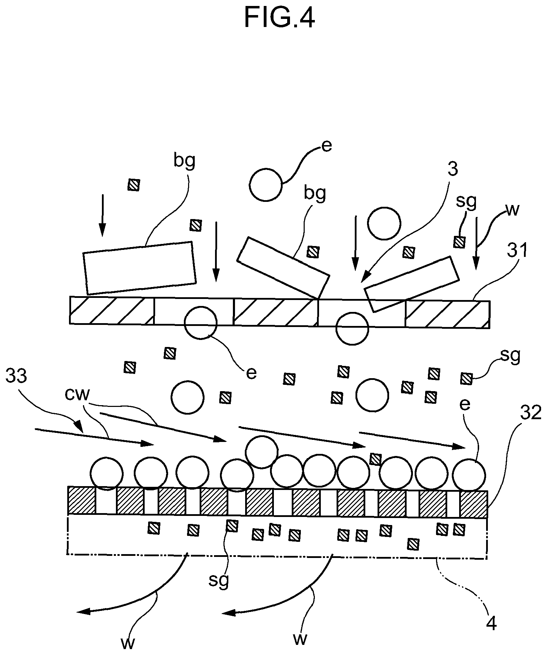

As specifically illustrated in FIG. 4, the unnecessary-material separating unit 3 includes a first net device 31 that allows the fish eggs e to pass therethrough and collects and removes unnecessary materials bg larger than the fish eggs e, a second net device 32 that is supported by the vibration conveying unit 4 and has a mesh not allowing the fish eggs e to pass therethrough, and a clean-water ejection unit 33 that ejects clean water cw to the fish eggs e in the second net device 32. In the present embodiment, a configuration is employed in which the breeding water w that has passed through the second net device 32 or has been crushed by collision with the second net device 32 is further separated from small unnecessary materials sg that is, for example, smaller than the fish eggs e and has passed through the second net device 32, and then is introduced again into the breeding water tank B. Further, in the present embodiment, the clean-water ejection unit 33 ejects the clean water cw to the fish eggs e in a direction in which the fish eggs e are conveyed by the vibration conveying unit 4 to convey the fish eggs e more quickly. That is, in the present embodiment, a configuration is employed in which the breeding water w that has passed through the second net device 32 and may be contaminated with bacteria, microorganisms, and the like is not introduced into the processing device disposed in the processing water tank 8. Here, the clean water cw in the present embodiment refers not only to distilled water and tap water but also to water that is less contaminated than the breeding water w.

The vibration conveying unit 4 guides the fish eggs e to the processing water tank 8 by applying predetermined vibration to the second net device 32. The fish eggs e to which the vibration is applied by the vibration conveying unit 4 are conveyed and put into the processing water tank 8 quickly and efficiently.

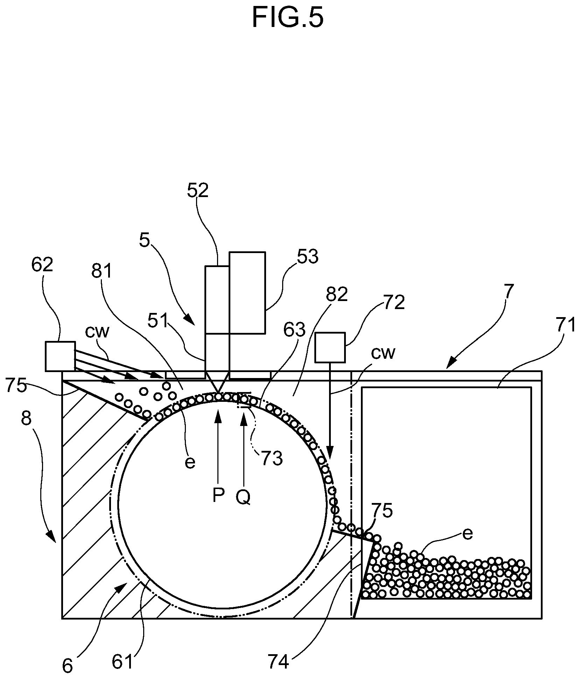

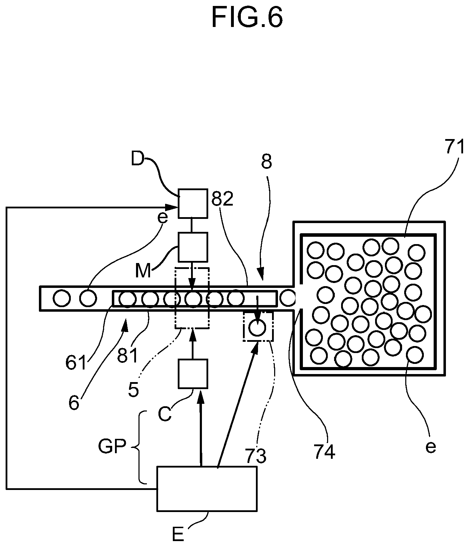

Here, as illustrated in FIGS. 5 and 6, in the present embodiment, the gene injection unit 5, the aligning and conveying unit 6, and the selection and collection unit 7 are provided in the processing water tank 8. Specifically, the gene injection unit 5 is disposed above the processing water tank 8, and the aligning and conveying unit 6 and the selection and collection unit 7 are disposed inside the processing water tank 8. As illustrated in FIGS. 2 and 5, water is filled up to a vicinity of an upper end of the processing water tank 8.

The processing water tank 8 performs processing on the collected fish eggs e, and is provided with the gene injection unit 5 and the aligning and conveying unit 6 which are processing units that inject a predetermined substance into the fish eggs e. More specifically, the processing water tank 8 includes a pre-processing region 81 in which an internal thickness of the processing water tank 8 is sized so that a plurality of fish eggs e cannot be arranged in parallel to house the fish eggs e before processing, and a post-processing region 82 for housing the fish eggs e to which the predetermined processing has been performed. More particularly, the processing water tank 8 has a substantially T-shape in a plan view in which the pre-processing region 81 in which the internal thickness is sized so that a plurality of fish eggs e cannot be arranged in parallel, and the post-processing region 82 are continuous. In the present embodiment, at least a portion at a processing position P is formed of a transparent material to visually recognize at least the processing position P.

The aligning and conveying unit 6 aligns the fish eggs e introduced into the pre-processing region 81 so that the gene injection unit 5 easily perform processing. The aligning and conveying unit 6 includes an alignment board 61 shaped in a disc body and provided with a plurality of housing recesses 63 formed at a predetermined pitch around the disc at an equal interval, and an aligning pump 62 that ejects the clean water cw to the alignment board 61. The fish eggs e introduced into the pre-processing region 81 are smoothly guided to the alignment board 61 by the clean water cw from the aligning pump 62 and positioned in each of the housing recesses 63. The alignment board 61 corresponds to a placing unit on which the fish eggs e can be placed. In the present embodiment, as illustrated in FIG. 6, the rotation of the alignment board 61 is precisely controlled by driving an AC servomotor M via a motor driver D. The motor driver D is controlled by a control device E. As long as the fish eggs can be arranged and conveyed one by one, for example, the alignment board 61 may be a belt-like or chain-like component that can form an endless track.

The gene injection unit 5 injects the gene solution G into each of the fish eggs e conveyed by the aligning and conveying unit 6, and includes a capillary 51 having a substantially needle shape that directly injects a gene into each of the fish eggs e, a syringe pump 52 that supplies a predetermined amount of genes to the capillary 51, and a positioner 53 that positions the capillary 51 and the syringe pump 52 in a vertical direction (Z direction). The positioner 53 also positions the capillary 51 and the syringe pump 52 in XY directions. The capillary 51 corresponds to the introduction tube having a tube shape that can introduce a predetermined substance into each of the fish eggs e at a predetermined timing.

In the present embodiment, there is provided the selection and collection unit 7 that efficiently collects the fish eggs e into which the gene has been injected, from the aligning and conveying unit 6 to the post-processing region 82.

The selection and collection unit 7 corresponds to a collection device or a collection tank that collects the processed fish eggs e. The selection and collection unit 7 includes a collection container 71 serving as a collection tank accommodated in the post-processing region 82, a collection pump 72 that is provided near the post-processing region 82 in the pre-processing region 81 and ejects the clean water cw to the alignment board 61, a sorting unit 73 that discharges the fish egg e, to which a gene has not been accurately introduced by the gene injection unit 5 included in the processing device, out of the processing water tank 8 through an NG egg exclusion path, and a guide 75 that is provided to cover the lower half of the alignment board 61 in the processing water tank 8 and guides the fish eggs e from the pre-processing region 81 to an OK egg collection path leading to the post-processing region 82. The OK egg collection path is provided along the guide 75. The collection container 71 according to the present embodiment is provided with a slit 74 in a portion facing the post-processing region 82, and thus, can efficiently introduce the fish eggs e moved from the pre-processing region 81 into the collection container 71. Further, in the present embodiment, a part of the guide 75 is formed to enter into the collection container 71, and thus, the fish eggs e are efficiently guided to the collection container 71.

The sorting unit 73 includes a water flow urging pump for exclusion, an urging water input and output tube serving as the NG egg exclusion path, and a solenoid valve that opens and closes the tube. When the housing recess 63 housing the eggs e determined as NG are sent to an exclusion position, the solenoid valve is opened, and the eggs e are urged by water flow to be removed from the housing recess 63, and then sent out to the urging water input and output tube.

Here, in the present embodiment, as illustrated in FIGS. 5, 6, and 7, a configuration is employed in which the fish eggs e introduced into the processing water tank 8 are processed with higher efficiency. That is, the fish eggs e introduced into the pre-processing region 81 in the processing water tank 8 are smoothly guided to the housing recess 63 by the clean water cw ejected from the aligning pump 62. The fish eggs e housed in the housing recess 63 move to a position immediately below the capillary 51 by rotation of the alignment board 61 without changing the order. Here, the width in a direction perpendicular to the paper surface of a portion toward the pre-processing region 81 and the OK egg collection path is, including the processing position P, sized so that two or more fish eggs e cannot be arranged, and the portion toward the pre-processing region 81 and the OK egg collection path is, including the processing position P, formed of a transparent member. That is, the fish eggs e guided to the position immediately below the capillary 51 are always clearly visible one by one. The capillary 51 is arranged, for each of the fish eggs e, in a state where a position of the embryo inside the fish egg e can be clearly visually recognized by a camera C illustrated in FIG. 6, and thus, it is possible to accurately inject a gene into the embryo while the camera C and the gene injection unit 5 are precisely controlled. At this time, the alignment board 61 is formed in a substantially circular shape and the recess 63 is arranged at equal intervals, and thus, the recess 63 is accurately positioned directly below the capillary 51 by the control device E. Here, if each of the fish eggs e is not suitable for gene injection or if it is visually recognized by the camera C that the gene could not be injected accurately, the fish egg e can be separately collected by allowing the fish egg e to pass through the sorting unit 73 provided at a position where the alignment board 61 is rotated by a predetermined angle. This prevents eggs that may have undergone recombination from unnecessarily leaking out. At the same time, the fish egg e into which the gene has been accurately injected is guided at a high rate downstream of the post-processing region 82. Further, the fish eggs e into which the gene has been injected by the capillary 51 is reliably separated from the housing recess 63 by the clean water cw ejected from the collection pump 72. These fish eggs e are smoothly guided from the post-processing region 82 to the OK egg collection path, pass through the slit 74, and are housed in the collection container 71.

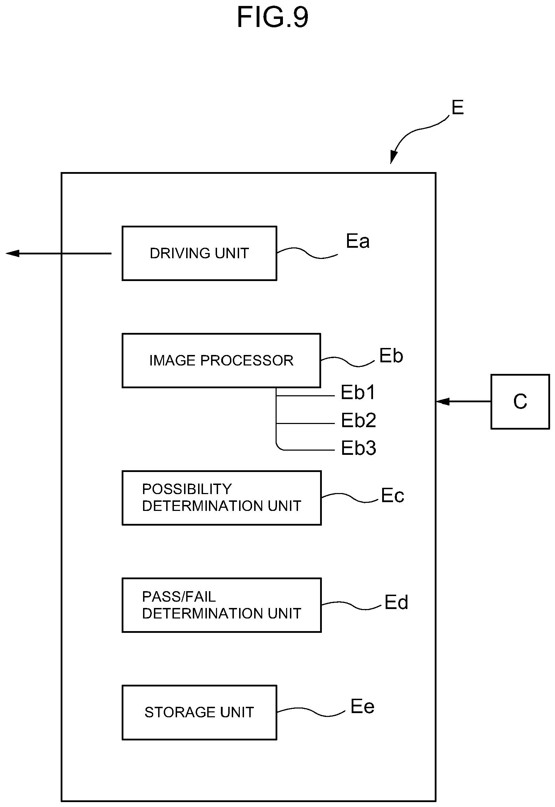

The above-described camera C constitutes an optical axis system for imaging fish eggs in a direction that is orthogonal to a conveyance direction of the housing recess 63 and an elevating direction, and the position of the camera C can be adjusted in the XYZ directions through a driving unit Ea of the control device E illustrated in FIG. 9, together with the above-described positioner 53.

The control device E drives the sorting unit 73 and the positioner 53 through the driving unit Ea, and includes an image processor Eb that performs image processing on an image obtained by capturing inside the housing recess 63, a possibility determination unit Ec that determines whether the each of the fish eggs e is suitable for gene injection, and a pass/fail determination unit Ed that determines through the image processing after the injection whether each of the fish eggs e is properly injected.

In the possibility determination, an image is captured by the camera when the fish eggs have settled after a predetermined time has elapsed since a stroke operation of the capillary 51 serving as the introduction tube. In the pass/fail determination, the gene solution to be injected is colored with a pigment such as phenol red (red). In the pass/fail determination through the image processing, the colored color is detected to determine pass or fail.

The image processor Eb includes an edge detection filter unit Eb1 that detects contours of a concave portion, an egg membrane, an embryonic membrane, and the like, based on the brightness, or the like of the image captured by the camera C, a template unit Eb2 that stores reference data on the concave portion, the egg membrane, the embryonic membrane, and the like, and a matching unit Eb3 that performs image matching between the data of the template unit Eb2 and the detected edge. The image processor Eb performs image matching according to requests from the possibility determination unit Ec and the pass/fail determination unit Ed, and returns a result. The camera C illustrated in FIG. 6 constitutes an image processing unit GP together with the image processor Eb.

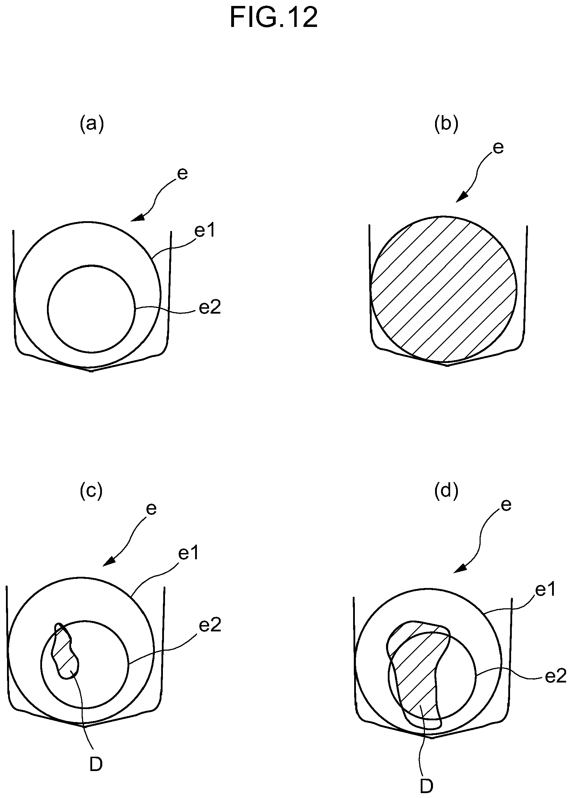

The possibility determination unit Ec defines, as conditions for "OK" to injection the gene solution, a case where the housing recess 63 of the alignment board 61 is within a predetermined range of the image (condition 1), and as illustrated in FIG. 12(a), a case where one egg membrane e1 (outer membrane) and one embryonic membrane e2 (inner membrane) are detected within an appropriate range for the capillary 51 to pierce (condition 2). For this purpose, a permissible deviation amount, a deviation amount of the embryonic membrane, and the like are stored as the reference data, and the possibility determination is made based on the result from the image processor Eb.

For the condition 2, a case where no fish egg is placed, a case where the fish egg is dead (see FIG. 12(b)), a case where the fish egg is damaged (there is no egg membrane or no embryo), and the like are excluded. According to the condition 1, a case where the alignment board cannot be correctly positioned, a case where an illumination is not turned on, and the like are excluded.

The pass/fail determination unit Ed defines, as conditions for an injection result being "compatible," a case where one egg membrane e1 (outer membrane) and one embryonic membrane e2 (inner membrane) are each detected within the predetermined range of the image (condition 3), a case where the number of pixels of pigment detected in the egg membrane e1 (hatched portions in FIGS. 12(c) and 12(d)) is within a predetermined range (condition 4), and a case where a ratio of pigment detected in the embryo e2 among the pigment detected in the egg membrane e1 is equal to or higher than a predetermined value (condition 5). Thus, a predetermined range permissible for the egg membrane e1 or the embryonic membrane e2, a threshold value of the number of pixels of pigment detected in the egg membrane e1, and a threshold value of the ratio of the pigment detected in the embryo e2 are stored as the reference data, and the pass/fail determination is made based on a result obtained through the image processor Eb.

For the condition 3, a case where a fish egg is damaged by gene injection is excluded, and for the condition 4, a case where the gene solution has not been injected into the embryo and a case where the amount of injection is large or small are excluded, and for the condition 5, a case where most of the gene solution has leaked from the embryo after the injection, a case where the gene solution is injected between the egg membrane e1 and the embryo e2, a case where the gene solution is injected in a position deviated in an optical axis direction, and the like are excluded.

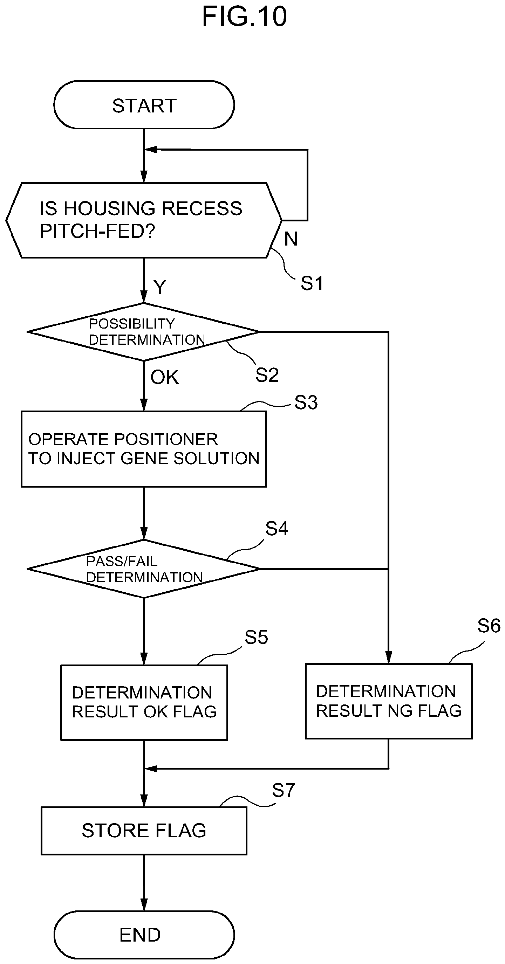

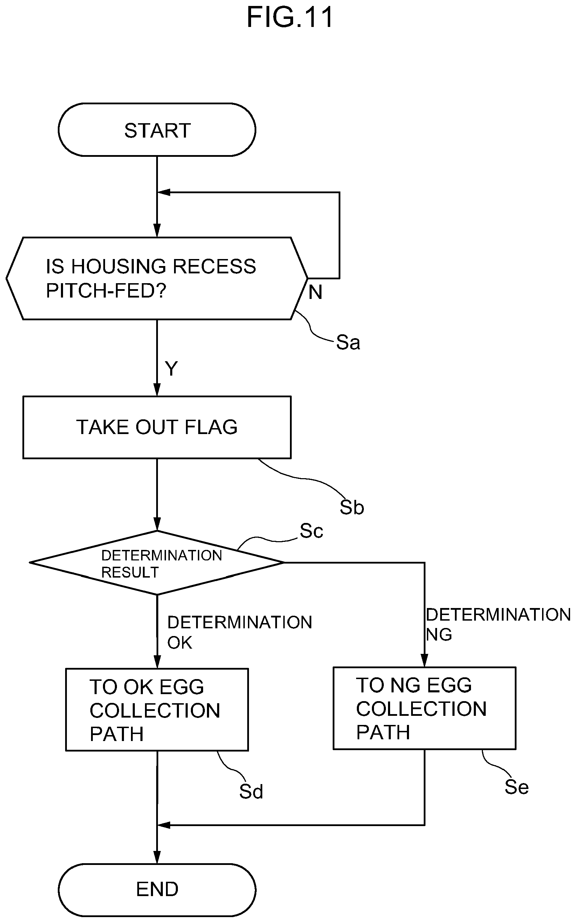

The possibility determination unit Ec and the pass/fail determination unit Ed illustrated in FIG. 10 determine the fish eggs through a determination flow illustrated in FIG. 10, and sort the fish eggs into OK eggs and NG eggs through a processing flow illustrated in FIG. 11.

Firstly, in the determination flow, it is determined whether the housing recess 63 has been pitch-fed to the processing position (step S1). In the case of YES, it is determined whether to inject a gene to a fish egg e to be injected with the gene, through the possibility determination unit Ec (step S2). If the determination result is OK, the gene solution is injected by raising and lowering the positioner 53 (step S3). Further, whether the injection result is compatible is determined on the fish egg e into which the gene has been injected, through the pass/fail determination unit Ed (step S4). If the determination result is OK, a determination OK flag serving as a determination result flag is turned on (step S5). Conversely, if the determination result is NO in steps S2 and S4, a determination NG flag serving as the determination result flag is turned on (step S6). The determination result is stored in a storage unit Ee (step S7), and the processing ends.

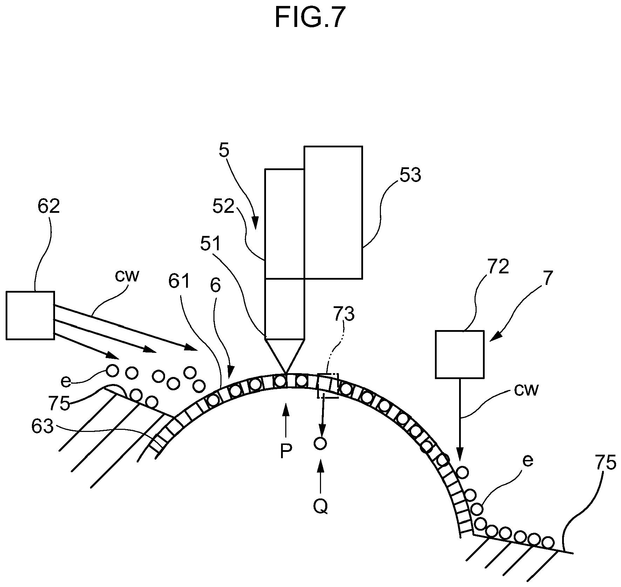

On the other hand, in the processing flow illustrated in FIG. 11, it is determined whether the housing recess 63 has been pitch-fed to the sorting position (step Sa). In the case of YES, a determination result obtained a predetermined number of pitches before is taken out from the storage unit Ee (step Sb). As illustrated in FIG. 7, a position Q of the sorting unit 73 is set at a position away from the processing position P at which the gene is injected, and the housing recess 63 reaches the sorting position Q after moving a predetermined number of pitches from the processing position P, and thus, in step S7, the storage unit Ee buffers at least the preceding determination result flag so that the preceding determination result is taken out. For example, if the processing position P and the sorting position Q are separated by three pitches, four determination result flags are buffered.

Next, it is determined whether the determination is OK or NG (step Sc). In the case of YES, the fish egg e is caused to flow into the OK egg collection path (step Sd), and in the case of NO, the sorting unit 73 is operated to flow the fish egg e into an NG egg collection path (step Se), and the processing ends.

Thus, within one tact time, the possibility determination and the pass/fail determination on the fish eggs e at the processing position P and the sorting processing of the previously determined fish eggs at the sorting position Q are performed in parallel. The above-described possibility determination and pass/fail determination include a waiting time for waiting for the settlement of the fish eggs e after the pitch-feeding of the alignment board 61 and the operation of the positioner, an image capturing time, an image processing time for determination, and the like.

It is noted that in the alignment board 61 including the housing recess 63, an error in assembling to a motor is inevitable, and thus, a shaft center of the motor may not coincide with a shaft center of the alignment board 61. Thus, when the alignment board 61 is rotated, the position of the housing recess 63 arranged at the processing position P is not necessarily fixed even if the housing recess 63 can be processed highly accurately to maintain the same shape as that of another housing recess 63. The alignment board 61 pitch-feeds at a predetermined angle from the once determined initial position and does not perform feedback control for positioning the housing recesses 63 at the processing position P every time the alignment board 61 pitch feeds, and thus, even though the capillary 51 is accurately positioned, lowered, and raised, it is inevitable that the housing recesses 63 that have reached the processing position P are slightly displaced from each other.

Therefore, the image processing unit GP is configured to set, as a reference point X, a relative position at which a front end 51a of the capillary 51 to be positioned with respect to the housing recess 63, and automatically acquire a coordinate of a standardized reference point X, based on reference points X in the plurality of housing recesses 63 imaged while the alignment board 61 is rotated.

Specifically, in the present embodiment, an edge shape of the housing recess 63 is stored in advance as template data, and a reference point is set to a front end position at which the capillary 51 is to be introduced in the housing recess 63. Next, a process of acquiring the coordinate of the reference point X in the imaged housing recess 63 is performed for all or some of the housing recesses 63 for one round, through an image matching between the housing recess 63 imaged as illustrated in FIG. 11 and the housing recess of the template data. As a result, an initial teaching and the like through the positioner 53 illustrated in FIG. 5 can be provided by using an average coordinate of the reference point X as the front end position of the capillary 51 to be actually introduced.

As described above, the gene injection apparatus 1 being a fish egg processing apparatus according to the present embodiment includes the processing water tank 8 to which fish eggs are guided, the aligning and conveying unit 6 that conveys, to the predetermined processing position P, the housing recess 63 that houses the fish eggs e one by one in the processing water tank 8, the gene injection unit 5 serving as a processing unit that raises and lowers the capillary 51 serving as an introduction tube to inject the gene solution G as a predetermined substance into the fish eggs e in the housing recess 63 conveyed to the processing position P, the image processing unit GP that images the housing recess 63 from the direction that intersects the depth direction and the conveying-in direction,-in direction in association with the processing water tank 8, and the possibility determination unit Ec that determines whether to inject the predetermined substance into each of the fish eggs before processing, based on an image obtained through the image processing unit GP and a previously determined condition. The injection operation of the gene injection unit 5 is controlled based on the determination result of the possibility determination unit Ec.

Thus, even if the in-line system is employed, it is possible to prevent the processing from being performed in an improper state not satisfying the conditions or being performed on an improper fish egg.

Further, the predetermined condition includes conditions that: the housing recess 63 is within a predetermined range with respect to the gene injection unit 5; and one egg membrane and one embryonic membrane are detected within a predetermined range in the housing recess, and thus, it is possible to reliably exclude a case where the housing recess is not correctly positioned or a case where the fish egg is not placed, from fish eggs to be processed.

Alternatively, the gene injection apparatus 1 being a fish egg processing apparatus according to the present embodiment includes the processing water tank 8 to which fish eggs are guided, the aligning and conveying unit 6 that conveys, from the predetermined processing position P, the housing recess 63 that houses the fish eggs e one by one in the processing water tank 8, the gene injection unit 5 serving as a processing unit that raises and lowers the capillary 51 serving as an introduction tube to inject a predetermined substance into each of the fish eggs e in the housing recess 63 at the processing position P, the sorting unit 73 that determines a conveyance destination according to the quality of the fish eggs e, the image processing unit GP that images the housing recess 63 from the direction that intersects the depth direction and the conveying-out direction, in association with the processing water tank 8, and the pass/fail determination unit Ed that determines whether each of the processed fish eggs is suitable for collection, based on an image obtained through the image processing unit GP and a previously determined condition. The sorting operation of the sorting unit 73 is controlled based on the determination result of the pass/fail determination unit Ed.

Thus, even if the in-line system is employed, it is possible to remove fish eggs to which the predetermined substance is injected improperly without satisfying the conditions.

In addition, the gene solution contains pigment at a time of injection in the gene injection unit5, and the predetermined condition include conditions that: one egg membrane e1 and one embryonic membrane e2 are detected within a predetermined range in the image; the number of pixels of pigment detected in the egg membrane e1 is within a predetermined range; and the ratio of pigment detected in the embryo e2 among the pigment detected in the egg membrane e1 is equal to or higher than a predetermined value, and thus, it is possible to reliably exclude a fish egg damaged by the injection, a fish egg of which injection state is improper, or a fish egg from which the injected substance is leaked out.

The gene injection apparatus 1 includes the storage unit Ee that stores the determination result of the determination units Ec and Ed at the processing position P for each pitch while the housing recess 63 is pitch-fed, and, when the sorting unit 73 located a predetermined number of pitches away from the processing position P is controlled, takes out, from the storage unit Ee, a determination result obtained a predetermined number of pitches before to control the sorting operation of the sorting unit 73 based on the determination result.

Thus, even if the processing position P is separated from the sorting position Q, the determination process and the sorting process can be performed in parallel.

Further, the aligning and conveying unit 6 includes the alignment board 61 in a gear shape provided with the housing recess 63 in the whole circumference. The plurality of housing recesses 63 are imaged by the image processing unit GP, and the reference point X is standardized (averaged), in which the reference point X is a relative position of the capillary front end 51a and is set as a preferable position in each of the housing recesses 63.

Thus, the influence due to assembling errors of the alignment board 61 is minimized, and even if the predetermined substance is injected by raising and lowering the introduction tube while the housing recess is pitch-fed in an initially positioned state, it is possible to effectively avoid a situation where poor injection occurs or a needle breaks as a result of contact with the alignment board.

Although the embodiment of the present invention has been described above, the present invention is not limited to the configuration of the above-described embodiment. For example, in the above-described embodiment, both the possibility determination and the pass/fail determination are performed, but this does not preclude a simplified configuration to perform only the possibility determination or a simplified configuration to perform only the pass/fail determination.

Although the mode for introducing a gene into a fish egg is disclosed in the above-described embodiment, it is obviously possible to adopt a mode for injecting, into a fish egg, different substances from genes, for example, cells such as human cancer cells, drugs, drug candidate substances, chemical substances such as toxic substances, or food additives such as seasonings or coloring agents. Further, in the above-described embodiment, a zebrafish egg is applied as a fish egg, but, of course, an egg of another fish may be employed. Further, the specific arrangement of individual constituent components, including a detailed mode such as specific water flow in the processing water tank and other configurations, can be variously modified within the scope of the present invention.

### INDUSTRIAL APPLICABILITY

The present invention can be used as a fish egg processing apparatus for performing predetermined processing mainly on fish eggs.

### [Reference Signs List]

- 1 ...: Fish egg processing apparatus (gene injection apparatus)
- 5 ...: Processing unit (gene injection unit)
- 6 ...: Aligning and conveying unit
- 8 ...: Processing water tank
- 51 ...: Introduction tube (capillary)
- 51a ...: Front end
- 63 ...: Housing recess
- 73 ...: Sorting unit
- e ...: Fish egg
- e1 ...: Egg membrane
- e2 ...: Embryonic membrane
- Ec ...: Possibility determination unit
- Ed ...: Pass/fail determination unit
- Ee ...: Storage unit
- GP ...: Image processing unit
- X...: Reference point

## Claims

1. A fish egg processing apparatus, comprising:
a processing water tank to which fish eggs are guided;
an aligning and conveying unit that conveys, to a predetermined processing position, a housing recess that houses the fish eggs one by one in the processing water tank;
a processing unit that raises and lowers an introduction tube to inject a predetermined substance into each of the fish eggs in the housing recess conveyed to the processing position;
an image processing unit that images the housing recess from a direction that intersects a depth direction and a conveying-in direction, in association with the processing water tank; and
a possibility determination unit that determines whether to inject the predetermined substance into each of the fish eggs before processing, based on an image obtained through the image processing unit and a previously determined condition, wherein
an injection operation of the processing unit is controlled based on a determination result of the possibility determination unit.

2. The fish egg processing apparatus according to claim 1, wherein the predetermined condition includes at least one or both of conditions that: the housing recess is within a predetermined range with respect to the processing unit; and one egg membrane and one embryonic membrane are detected within a predetermined range in the housing recess.

3. A fish egg processing apparatus, comprising:
a processing water tank to which fish eggs are guided;
an aligning and conveying unit that conveys, from a predetermined processing position, a housing recess that houses the fish eggs one by one in the processing water tank;
a processing unit that raises and lowers an introduction tube to inject a predetermined substance into each of the fish eggs in the housing recess at the processing position;
a sorting unit that determines a conveyance destination according to a quality of each of the fish eggs;
an image processing unit that images the housing recess from a direction that intersects a depth direction and a conveying-out direction, in association with the processing water tank; and
a pass/fail determination unit that determines whether each of the processed fish eggs is suitable for collection, based on an image obtained through the image processing unit and a previously determined condition, wherein
a sorting operation of the sorting unit is controlled based on a determination result of the pass/fail determination unit.

4. The fish egg processing apparatus according to claim 3, wherein the predetermined substance contains pigment at the time of injection in the processing unit, and the predetermined condition includes at least one or two, or all of conditions that: one egg membrane and one embryonic membrane are detected within a predetermined range in the image; the number of pixels of pigment detected in the egg membrane is within a predetermined range; and a ratio of pigment detected in the embryo among the pigment detected in the egg membrane is equal to or higher than a predetermined value.

5. The fish egg processing apparatus according to any one of claims 1 to 4, comprising a storage unit that stores the determination result of the determination unit at the processing position for each pitch while the housing recess is pitch-fed, wherein when the sorting unit located a predetermined number of pitches away from the processing position is controlled, a determination result obtained a predetermined number of pitches before is taken out from the storage unit to control the sorting operation of the sorting unit based on the determination result.

6. The fish egg processing apparatus according to any one of claims 1 to 5, wherein the aligning and conveying unit includes an alignment board in a gear shape provided with the housing recess in the whole circumference, and
the image processing unit sets, as a reference point, a relative position at which a front end of the introduction tube is positioned with respect to the housing recess, and automatically acquires a coordinate of a standardized reference point, based on reference points in a plurality of housing recesses imaged while the alignment board is rotated.
